# EUROPEAN PATENT APPLICATION

(11) **EP 3 132 772 A1**
(43) Date of publication of application: **22.02.2017**
(21) Application number: 15181945.5
(22) Date of filing: 21.08.2015
(51) Int. Cl.: A61F 2/12

(54) **BREAST IMPLANT FOR PREVENTION AND TREATMENT**

(71) Applicant: Mahler, Marc, 10115 Berlin (DE)
(72) Inventor: Mahler, Marc, 10115 Berlin (DE)
(74) Representative: Rüger, Barthelt & Abel

(57) **Abstract**

A breast implant (1) comprising a shell (3) enclosing an internal chamber (2) configured to be substantially filled with a filler composition, wherein the implant comprises a channel (4) arranged inside the shell for guiding a fluid, and comprising at least one inlet opening (6) for injecting the fluid into the channel , and at least one outlet opening (5) for releasing the fluid out of the channel, wherein the at least one outlet opening is arranged in a wall of the shell for releasing the injected fluid to the outside of the implant.

## Description

The present invention relates to a breast implant, a method for the treatment or prevention of a breast implant-associated disease and a method for manufacturing such implant.

A breast implant is a prosthesis used to change the size, form, and texture of a breast, particularly a woman's breast. In plastic surgery, emplacement of breast implants is mainly applied for three purposes, i.e. for post-mastectomy breast reconstruction, where breast tissues damaged by trauma (blunt, penetrating, blast), disease (breast cancer), and failed anatomic development (tuberous breast deformity). Missing or damaged breast is re replaced by the breast implant. This is for revision and reconstruction, e.g. to revise (correct) the outcome of a previous breast reconstruction surgery or for correcting congenital defects and deformities of the chest wall. Also, implants are used for aesthetic breast augmentation, i.e. to aesthetically augment the size, form, and feel of the breasts. In the United States about 300,000 women per year underwent cosmetic implantation, whereas about 90,000 to 100,000 women per year underwent breast reconstruction implantation, see Marques et al.; 2010; Long-term follow-up of breast capsule contracture rates in cosmetic and reconstructive cases; Plastic and Reconstructive Surgery, 126(3):769-78.

Breast implants known in the art basically consist of a shell, usually an elastomeric silicone shell, which encloses an internal chamber filled with a suitable sterile filler composition, e.g. saline solution, viscous silicone gel, or composite filler. Moreover, particularly for the reconstruction of a breast, in surgical practice a tissue expander is used as a temporary breast implant to form and establish an implant pocket for emplacing the permanent breast implant. For this purpose the breast implant comprises a filling port, which is used to inject the filler composition, usually saline solution, into the expander and thus expand and form the implant pocket.

The plastic surgical emplacement of breast implants basically presents the same health risks common to surgery, such as adverse reaction to anesthesia, hematoma, late hematoma, seroma, incision-site breakdown, and the like. However, a major post-emplacement complication represents unfavorable scarring, particularly capsular contracture. Capsular contracture occurs when the collagen-fiber capsule tightens and squeezes the breast implant; as such, it is a medical complication that can be very painful and discomforting, and might distort the aesthetics of the breast implant and the breast.

The cause of capsular contracture is unknown, however, it is believed that the disease is manifested as a response of the human body's immune system to a surgically installed foreign object, i.e. for example a breast implant. The implant is encapsulated with scar tissue capsules of tightly woven collagen fibers, in order to maintain the integrity of the body by isolating the foreign object and so tolerate its presence. The common incidence factors for the occurrence of capsular contraction include bacterial contamination, rupture of implant shell, leakage of the filler composition, and hematoma. The surgical implantation procedures that have reduced the incidence of capsular contracture include sub-muscular emplacement, the use of breast implants with a textured or polyurethane-coated surface; limited pre-operative handling of the implants, limited contact with the chest skin of the implant pocket before the emplacement of the breast implant, and irrigation of the recipient site with triple-antibiotic solutions.

Statistically, about 5 to 20% of women who underwent cosmetic implantation and about 40 to 55% of women who underwent breast reconstruction implantation developed capsular contracture. As a result, in the latter case physicians even started to advice against implantation for this reason, among others.

Non-surgical methods of treating capsules are known and include massage, external ultrasound, treatment with leukotriene pathway inhibitors (e.g. Accolate®, Singulair®), and pulsed electromagnetic field therapy. The treatment of capsular contraction, however, is extremely difficult and the correction of capsular contracture often requires surgical incision (release) or removal of the capsule, so-called open capsulotomy or capsulectomy. The latter usually requires the removal and possible replacement of the breast implant itself. Closed capsulotomy, i.e. disrupting the capsule via external manipulation, a once-common maneuver for treating hard capsules, was discontinued because it might rupture the breast implant, led to significant pain and discomfort during the procedure and foremost revealed a very low success rate as well as high recurrence rate of capsular contracture.

Moreover, because capsular contracture is a consequence of the immune system defending the patient's bodily integrity and health, capsular contracture might reoccur even after the requisite corrective surgery for the initial incidence.

The Collagenase of the bacterium *clostridium histolyticum* is an enzyme that digests collagen. It is used as a powder-and-solvent injection kit for the treatment of Dupuytren's contracture, a condition where the fingers bend towards the palm and cannot be fully straightened, and Peyronie's disease, a connective tissue disorder involving the growth of fibrous plaques in the soft tissue of the penis. Recent studies proved that - in principle - collagenase enzyme is also useful to enzymatically dissolve the collagen-fiber capsule in capsular contracture. An unsolved problem, however, exists in how to apply the collagenase enzyme to the collagen capsule in a clinical setting.

US 2008/0183124 A1 describes a method and a surgical instrument for treating breast implant encapsulation. For this purpose, the instrument comprises a round blunt tip and an electrode at its distal end to create an incision into the skin and approach a breast implant underneath the incision. A fibrin matrix and enzyme collagenase is then introduced in the area near the implant to prevent or remove scar encapsulation. However, the method and instrument disclosed in this document allows the application of the enzyme only after a relatively severe surgical and thus invasive surgical procedure. Moreover, the site of the enzyme application is limited to the area underneath the incision.

It is an object of the present invention to provide a device and/or method, which allows for a less invasive possibility to apply a fluid, particularly a drug, to the tissue surrounding a breast implant. It is a further object of the present invention to provide a device and/or method, which allows a collagenase treatment for the effective prevention or therapy of capsular contracture.

These and other problems are solved by the subject matter of the attached independent claims.

The above objects of the invention are achieved by a breast implant according to claim 1, a method for the treatment or prevention of a breast implant-associated disease according to claim 13 and a method for manufacturing an implant according to claim 15. Preferred embodiments may be taken from the dependent claims.

A breast implant according to claim 1 comprises at least:
a shell (3) enclosing an internal chamber (2) configured to be substantially filled with a filler composition, wherein the implant (1) comprises a channel (4) arranged inside the shell (3) for guiding a fluid, and comprising at least one inlet opening (6) for injecting the fluid into the channel (4), and at least one outlet opening (5) for releasing the fluid out of the channel (4). In the breast implant (1) according to the present invention the at least one outlet opening (5) is arranged in a wall of the shell (3) for releasing the injected fluid to the outside of the implant (1).

Such a breast implant according to the present invention enables to apply a fluid to the tissue surrounding the surgically emplaced breast implant in a relatively non-invasive way. At least the implant allows for application by minimum invasive techniques. The fluid can comprise an active pharmaceutical ingredient (API), like collagenase enzyme and/or other drugs. For purposes of the present invention API and drug are used synonymous.

Particularly, the present invention allows applying a collagenase treatment for the effective prevention or therapy of capsular contracture for women with breast implant.

For this purpose, a fluid, particularly a fluid comprising a drug, like for example collagenase enzyme, is injected into the implant (1) into the channel (4) through the inlet opening (6) such that the channel (4) is filled with the fluid. Such injection is possible in a relatively non-invasive way, for example using a suitable injection device, e.g. a syringe (8), filled with the fluid and/or drug. The thus injected fluid is pressed through the channel (4) towards the outlet opening (5), which is arranged in a wall of the shell (3). Thereby, the channel (4) provides a closed system for guiding the fluid inside the shell (3) to the at least one outlet opening (5). The fluid is released through the outlet opening (5) directly to the area at the outside of the implant (1) surrounding said at least one outlet opening (5). In other words, the outlet opening (5) is fluidically connecting the at least one channel (4) with the outside of the implant (1), whereas the channel (4) is a fluidically tight and closed system towards the chamber (2) and the filler composition within said chamber (2).

Advantageously, the channel (4) is arranged inside the shell (3) and is thus not disturbing the outer surface of the implant (1). According to the present invention the fluid, and particularly the drug, may thus applied to the area at the outside of the implant (1) surrounding said at least one outlet opening (5) in a relatively easy and non-painful way. This is of particularly advantage for applying drugs and/or fluids to the surrounding tissue of a breast implant. Particularly for the prevention or treatment of disease, and more particularly of breast implant-associated disease, e.g. capsular contracture, the liquid and/or drug may thus be applied to the tissue surrounding the breast implant in an effective but minimum invasive way. It has to be noted that in capsular contracture the collagen tissue is usually surrounding the implant forming a capsule enclosing the implant. In such situation the implant according to the present invention allows for effective treatment of capsular contracture in that the liquid and/or drug is applied directly to the inner surface of the collagen capsule. Particularly, if a collagenase drug is applied the collagen capsule is enzymatically dissolved and may be distributed in the area between the capsule's inner surface and the outer surface of the shell (3) of the implant (1) according to the present invention.

In an embodiment of the breast implant (1) according to the present invention, the breast implant (1), particularly the chamber (2) is filled with filler composition prior to surgical emplacement. However, alternatively the breast implant (1), particularly the chamber (2), may also be filled with filler composition after surgical emplacement. For the latter purpose, the breast implant (1) according to the present invention may be provided as a tissue expander and may comprise an inlet for filling filler composition (11). A breast implant according to the present invention can be provided in the form of an expander (tissue expander or expandable implant). An expander needs an inlet for filler composition (11). Such an inlet can be arranged in a sequential manner to the inlet opening (6). Also, inlet (11) and inlet opening (6) can be arranged in a parallel manner. A tissue expander can serve as temporary breast prosthesis used to form and establish an implant pocket for emplacing a permanent breast implant.

As the chamber (2) of the breast implant (1) according to the present invention is fluid tight, the breast implant (1) may be filled with any filler composition. Preferably, the filler composition is selected from saline solution, viscous silicone gel, and composite filler.

The present inventor has surprisingly found that the breast implant (1) according to the present invention not only allows for the one-time application of the fluid or drug, but may also be used for a repeated application of a fluid, particularly a drug. Accordingly, the injection of the fluid may be repeated in a regular or irregular manner and thus allows for the effective application of a fluid and/or drug in a multiple application dosage regimen. Particularly, collagenase treatment may regularly or irregularly be applied for multiple times in order to effectively dissolve a collagen capsule formed around the implant or prevent the formation or re-formation of such capsule.

In an advantageous embodiment of the inventive implant (1) the at least one inlet opening (6) is arranged inside the shell (3). Such arrangement can be in different manners. The inlet port can be constructed such that it comprises a part (6a) and a part (6b). (6a) is suited for multiple penetration and (6b) is the part of the inlet port that connects the inlet (6) with the channel (4). (6a) is made from a material suited for multiple penetrations, for example by means of a syringe. Such material can, for example, be hard rubber. The part (6a) of the inlet port can be arranged within the shell (3) or can be arranged adjacent to the inner part of the shell (3).

In case the implant (1) is in the form of an expander, besides the inlet opening (6) an inlet for filling filler composition (11) is present. For the inlet (11) the same applies as for the inlet opening (6), i.e. it can be arranged inside the shell (3) or adjacent to the inner part of the shell (3).

This advantageously allows for an even and undisturbed surface of the shell (3) of the implant (1) and, moreover protects the inlet opening (6) and/or the inlet for filling filler composition (11) against unwanted damage. For injection of the fluid and/or drug the injection device is introduced through the shell (3) and into the inlet opening (6). In line with this invention the fluid can be applied, for example by means of a syringe (8) through the outer layer of the shell directly into the inlet opening (6) by penetration of (6a). It has been found by the inventors that surprisingly the shell allows such injection, i.e. a penetration, into the inlet opening.

In the alternative the penetration part of the inlet opening (6a) is located in the shell (3) so that it is not necessary to even penetrate the shell.

In a further advantageous embodiment of the inventive implant (1) the inlet opening (6) comprises an injection port (7).

Such injection port (7) advantageously allows for simplified injection of the fluid and/or drug into the inlet opening (6).

The skilled person will understand that an injection port (7) can be arranged adjacent to the inlet opening (penetration part, 6a) similar as (6b) is arranged to (6a). Similar as described above, (6a) can be within the shell (3) or adjacent to the inner surface of the shell (3) also in a scenario that an injection port (7) is present.

In the alternative the injection port (7) can be similar to the inlet port (6) as to a two part form, penetration part and bridging part. The penetration part, synonymous to injection site, is suited for repeated injections, particularly repeated puncturing. For example, the injection port (7) may comprise an injection site substantially made from hard rubber or other suited material. Such hard rubber injection site allows for repeated puncturing with a needle or syringe without getting leaky.

The port is as small as possible and inside the shell (3). Arranged in such a manner that it is advantageously neither visible nor touchable and hence does not disturb the a smooth feeling of the implant. Suited ports are known to the skilled person and are, for example, described in US 9,079,004 B2. For this purpose, also ports usually applied in the art for venous or arterial lines may be applied, such as described in US 8,257,325 B2.

The injection port (7) can be present in addition to the inlet opening (6) according to advantageous embodiments of the present invention. Then, the injection port (7) is a separate device. The injection port (7) can be similar as the inlet port (6) comprising the two portions (6a) and (6b) and hence can substitute the inlet opening (6).

Both devices (6) and (7) have are different from an inlet for filling filler composition (11).

In connection therewith it is also important to note that injection port (7) at the inlet opening (6) usually is not under pressure from the side of the channel (4). This is as the channel (4) prior to its first use is empty or filled in a pressure-neutral manner. By contrast the filling port for injection of the filler composition (11) in an expander usually is under pressure if the chamber (2) is filled with filler composition. As moreover, the channel (4) is fluidically tight, the injection port (7) is secure with regard to an unwanted leak out of the filler composition.

In a further advantageous embodiment of the inventive implant (1), the implant comprises one singular unbranched channel (4) having one singular outlet opening (5) and one singular inlet opening (6).

Such configuration of the implant (1) according to the present invention particularly allows for a relatively cost-effective manufacturing of the implant (1). Moreover, as the implant (1) comprises one singular outlet opening (5), it advantageously allows a targeted delivery of the fluid and/or drug to a single desired target side.

However, other configurations and with more than one outlet opening (5) are explicitly considered to be within the scope of the invention.

Particularly, in a further advantageous embodiment of the inventive implant (1), the implant (1) comprises a plurality of channels (4) and or a branched channel (4). Thereby, in an embodiment each of the plurality of channels (4) may represent a singular unbranched channel (4) having each one singular outlet opening (5) and one singular inlet opening (6). Such configuration advantageously allows a targeted delivery of the fluid and/or drug to more than one desired target side. Moreover, if one of such plurality of channels (4) may be blocked or clogged, another channel may be used for application of the fluid and/or drug. Additionally or alternatively a channel (4) according to the present invention may be a branched channel (4) with elements (4a), (4b) and (4c). Accordingly, one channel may be branched at least on one side, thus having more than one outlet opening (5).

For example, in an embodiment of the breast implant (1) said implant may comprise one singular channel (4) having one singular inlet opening (6) and being branched into three distal sub-channels each leading to one respective outlet openings (5a), (5b) and (5c).

Advantageously, an implant (1) having a branched channel (4) comprising more than one inlet opening (6) for injecting the fluid into the desired channel (4), allows the physician the choice and thus a more comfortable injection of the fluid and/or drug, and moreover, if one of such inlet openings (6) may be blocked or clogged, another inlet opening (6) may be used for application of the fluid and/or drug into the desired channel (4). Accordingly, in such embodiment of an implant (1) having more than one inlet opening (6), each inlet opening (6) may comprise a respective injection port (7).

Alternatively or additionally, an implant (1) having a branched channel (4) comprising more than one outlet openings (5) for releasing the fluid out of the desired channel (4), allows to release the fluid and/or drug simultaneously at more than one outlet opening (5). This particularly, allows for simultaneous treatment of more than one target side. Moreover, if one of such outlet openings (5) may be blocked or clogged, another outlet opening (5) may still be used for releasing the fluid out of the desired channel (4).

In a further embodiment of the inventive implant (1) the at least one inlet opening (6) and the at least one outlet opening (5) are located at distant, possibly opposite sides of the implant (1). Particularly, an outlet opening (5) may advantageously be located at the upper end of the implant (1). Inlet and outlet can also be arranged in such a manner that they are not opposite. Any fluid that is injected and leaves the outlet opening (5) can follow gravity and hence surround the implant. In an advantageous embodiment the fluid leaves the outlet opening (5) and is distributed over the outer surface of the implant (1) ending the up at the lowest part of the implant in relation to the upright human body.

The inlet opening (6) may advantageously be located at the lower lateral quadrant of the implant. It is advantageous that inlet opening (6) is placed subcutaneous rather than being covered by any muscle, like the pectoralis muscle.

Particularly, if the implant (1) is in the form of an anatomically formed implant, an outlet opening (5) may advantageously be located at the upper end of the implant (1), and/or the inlet opening (6) may advantageously be located lower than (5). The channel (4) can be straight or loopy.

As mentioned above, the present inventors have surprisingly found that locating at least one outlet opening (5) at the upper end regarding to the desired emplaced position of the implant (1) in the human body is advantageous as the released fluid and/or drug will be distributed or flow around the shell (3) due to gravity, particularly if the upper part of the human body is held in an upright position.

In a further advantageous embodiment of the inventive implant (1) the at least one outlet opening (5) comprises a closing mechanism. Such closing mechanism advantageously closes the outlet opening (5) temporarily and thus prevents clogging of the outlet opening (5), for example by caused by formation of scar tissue and/or capsule formation or re-formation. Particularly, such closing mechanism is openable, more particularly by injection of a respective fluid and/or drug into a respective inlet opening (6). In an embodiment such closing mechanism may be provided in the form of a plug, valve and/or valve flap. In an embodiment such closing mechanism may be provided in the form of a biochemical closing mechanism. For example, a biochemically dissolvable plug. Particularly, a collagen plug may be used as a biochemical closing mechanism, which may advantageously dissolved by injection of collagenase. Additionally or alternatively such closing mechanism may be provided in the form of a mechanical closing mechanism. For example, a valve and/or valve flap, which may allow release of the injected fluid and/or drug to the outside of the implant (1), due to injection pressure. Additionally or alternatively closing mechanism may be provided in the form of a silicon plug.

According to another embodiment of the present invention both, inlet opening (6) and outlet (5), are in a closed state prior to the first application of a fluid. In such a scenario the channel (4) is in a collapsed state prior to the first application of the fluid. With first application of a fluid through the inlet opening (6), optionally including a port (7), the port and/or the inlet opening are opened with the means, like a syringe, suited to administer the fluid. By means of pressure the fluid is guided through the channel (4) and ends up at the outlet (5), which is opened the first time due to the pressure of the fluid.

In an alternative embodiment the channel (4) is opened by means of a catheter. In such a scenario a syringe is used for first perforation of the inlet opening (6) and, if present, the port (7). Afterwards the channel (4) and the outlet opening are opened by the catheter prior to administration of a fluid which can comprise an API.

Preferred embodiments include substances in the material of the channel (4) which are suited to prevent clocking of the channel.

In alternative embodiments of the present invention the channel (4) is designed an located in such a manner that a catheter is suited to open a clocked channel prior to application of the fluid in case the channel (4) is indeed clocked. On the surface of suited catheters substances that prevent clocking of the channel (4), like heparin, can be present.

In a further advantageous embodiment of the inventive implant (1), the at least one channel (4) is formed as an integral part of the shell (3). This advantageously allows reducing the number of parts and may be produced in a cost-effective manner.

The shell (3) of the implant (1) according to the present invention is preferably manufactured from elastomer silicone. The channel (4) of the implant (1) according to the present invention is can be manufactured from elastomer silicone, too. Further materials are suited as long as such materials can be in a sterile state and do not disturb the haptic of the implant.

Preferably, were at least one channel (4) is formed as an integral part of the shell (3), the channel (4) and the shell (3) are manufactured from the same material, preferably elastomer silicone.

Alternatively, the shell (3) and the channel (4) of the implant (1) according to the present invention are formed as separate parts manufactured from the same or different material, preferably the same material, more preferably elastomer silicone.

A shell (3) of an implant (1) according to the present invention preferably comprises a smooth, textured or polyurethane-coated surface. Such textures are of different nature and well known in the art. For example, implants can be textured by means of small particles of silicone which are distributed onto the outer surface. The outer surface of the implant is not of importance to the implant (1) according to the invention.

In a further advantageous embodiment of the inventive implant (1) the at least one channel (4) is at least partially attached to the inner surface of the shell (3). This advantageously allows the more exact positioning of the channel (4) inside the shell (3). Such configuration is of particular advantage, if the channel (4) is formed as an integral part of the shell (3). It has to be understood that also in such configuration the channel (4) is attached to the shell (3) by the at least one outlet opening (5), which is arranged in a wall of the shell (3).

Alternatively, the channel (4) may be positioned loosely inside the shell (3). This can apply to the inlet (6) and a port (7), if present, too. In such scenarios the material in the shell of the implant that needs to be penetrated for application of the fluid into the inlet (6) or port (7) must be sufficiently tight to allow such penetration without any risk that filler composition can escape from the implant.

Independent therefrom the inlet opening (6) and/or the injection port (7) may be attached, integrated or embedded partly in/to the wall of the shell (3).

In a further advantageous embodiment of the inventive implant (1), the at least one channel (4) has an inner diameter of about 0.1 mm to about 5.0 mm. Particularly, the at least one channel (4) has an inner diameter of at least 0.1 mm, of at least 0.2 mm, of at least 0.25 mm, of at least 0.3 mm, of at least 0.4 mm, of at least 0.5 mm, of at least 0.75 mm, of at least 0.8 mm, of at least 0.9 mm, of at least 1.0 mm, of at least 1.5 mm, of at least 2.0 mm, of at least 2.5 mm, of at least 3.0 mm, of at least 3.5 mm, of at least 4.0 mm, of at least 4.5 mm, or of at least 4.75 mm. Particularly, the at least one channel (4) has an inner diameter of not more than 5.0 mm, of not more than 4.75 mm, of not more than 4.5 mm, of not more than 4.0 mm, of not more than 3.5 mm, of not more than 3.0 mm, of not more than 2.5 mm, of not more than 2.0 mm, of not more than 1.5 mm, of not more than 1.0 mm, of not more than 0.9 mm, of not more than 0.8 mm, of not more than 0.75 mm, of not more than 0.5 mm, of not more than 0.4 mm, of not more than 0.3 mm, of not more than 0.25 mm, or of not more than 0.2 mm. Particularly, the at least one channel (4) has an inner diameter of about 0.1 mm to about 5.0 mm, of about 0.1 mm to about 4.75 mm, of about 0.1 mm to about 4.5 mm, of about 0.1 mm to about 4.0 mm, of about 0.1 mm to about 3.5 mm, of about 0.1 mm to about 3.0 mm, of about 0.1 mm to about 2.5 mm, of about 0.1 mm to about 2.0 mm, of about 0.1 mm to about 1.5 mm, of about 0.1 mm to about 1.0 mm, of about 0.1 mm to about 0.9 mm, of about 0.1 mm to about 0.8 mm, of about 0.1 mm to about 0.75 mm, of about 0.1 mm to about 0.5 mm, of about 0.1 mm to about 0.4 mm, of about 0.1 mm to about 0.3 mm, of about 0.1 mm to about 0.25 mm, of about 0.1 mm to about 0.2 mm. Particularly, the at least one channel (4) has an inner diameter of about 0.1 mm to about 5.0 mm, of about 0.2 mm to about 5.0 mm, of about 0.25 mm to about 5.0 mm, of about 0.3 mm to about 5.0 mm, of about 0.4 mm to about 5.0 mm, of about 0.5 mm to about 5.0 mm, of about 0.75 mm to about 5.0 mm, of about 0.8 mm to about 5.0 mm, of about 0.9 mm to about 5.0 mm, of about 1.0 mm to about 5.0 mm, of about 1.5 mm to about 5.0 mm, of about 2.0 mm to about 5.0 mm, of about 2.5 mm to about 5.0 mm, of about 3.0 mm to about 5.0 mm, of about 3.5 mm to about 5.0 mm, of about 4.0 mm to about 5.0 mm, of about 4.5 mm to about 5.0 mm, or of about 4.75 mm to about 5.0 mm. Particularly, the at least one channel (4) has an inner diameter of about 0.1 mm, of about 0.2 mm, of about 0.25 mm, of about 0.3 mm, of about 0.4 mm, of about 0.5 mm, of about 0.75 mm, of about 0.8 mm, of about 0.9 mm, of about 1.0 mm, of about 1.5 mm, of about 2.0 mm, of about 2.5 mm, of about 3.0 mm, of about 3.5 mm, of about 4.0 mm, of about 4.5 mm, of about 4.75 mm, or of about 5.0 mm.

In a further advantageous embodiment of the inventive implant (1), the at least one inlet opening (6), particularly an injection port (7) if present, comprises a locating mechanism. This advantageously simplifies locating the inlet opening (6), and particularly an injection port (7), if present, from outside of the human body prior to injection. For example, such locating mechanism allows a physician to non-invasively locate the site of injection and subsequently target the inlet opening (6), and particularly an injection port (7), if present, with the injection device, e.g. a syringe. In an embodiment the locating mechanism may comprise or be partially manufactured from a ferromagnetic material. For example, such locating mechanism may be provided in the form of a metallic object, e.g. a metallic ring. Such locating mechanism comprising or being partially manufactured from a ferromagnetic material advantageously allows the detection and thus location through the skin of the human body using a simple magnet. Preferably, an injection port (7), if present, is surrounded by a metallic ferromagnetic ring element. However, it is also considered herein that in a patient were an MRT-scan is indicated or likely, for example, for cancer treatment and/or post-mastectomy breast reconstruction, using a metal locating mechanism may be disadvantageous. Therefore it is important to note that the at least one inlet opening (6), particularly an injection port (7) if present, may also be located by simple palpation. Alternatively or additionally, the at least one inlet opening (6), particularly an injection port (7) if present, may also be located by x-ray or ultrasound. For such a patient were an MRT-scan is indicated or likely, an implant (1) according to an embodiment of the present invention advantageously is manufactured solely from MRT-friendly materials.

The above described problems are also advantageously solved by a method for the treatment or prevention of a breast implant-associated disease according to claim 13.

Such method comprises at least the steps of:
- a) surgically emplacing a breast implant (1) according to the present invention,
- optionally, filling the implant (1) with filling composition,
- b) detecting the at least one inlet opening (6), and
- c) injecting a fluid comprising an effective dose of a drug into at least one inlet opening (6), or injection port (7), if present, until said fluid comprising the drug is provided through at least one outlet opening (5) to an area at the outside of the implant (1) surrounding said at least one outlet opening (5).
   The method for treatment by means of the implant
   Suited drugs for administration by means of the implant (1) according to the invention are, for example: antibiotics in case of infectious diseases, corticoids, for example for suppression of immune reaction in the case of autoimmune diseases or in the case of inflammatory diseases. Furthermore chemotherapeutic agents can be applied in cases cancer is involved.
   It becomes apparent that the implant according to the present invention allows minimal invasive application of drugs at the site the drug is needed. This avoids detrimental side effects that occur in case the drugs are administered orally or otherwise systemic. Administration of an API at the place it is needed provides several advantages. Small doses of API are sufficient. As mentioned before, side effects are minimized.

Such method advantageously allows the emplacement of the implant (1) according to the present invention in usual methods for emplacement of standard breast implants known to a person skilled in the art. However, advantageously the method for the treatment or prevention of a breast implant-associated disease according to the present invention also advantageously allows for a less invasive possibility to apply a fluid, particularly a drug to the tissue surrounding the emplaced breast implant. Particularly, said method allows for a repeated application of a fluid and/or drug. In an embodiment of the method for the treatment or prevention of a breast implant-associated disease according to the present invention, the method comprises repeating steps b) and c), whereby the fluid and/or drug may be same or different.

In an advantageous embodiment of the inventive method the breast implant-associated disease is capsular contracture, and wherein the drug comprises collagenase. Such method particularly allows a collagenase treatment for the effective prevention or therapy of capsular contracture.

The above described problems are also advantageously solved by a method according to claim 15 for manufacturing an implant (1) according to the present invention.

Such method comprises at least the steps of:
- providing a shell (3) enclosing an internal chamber (2) configured to be substantially filled with a filler composition,
- providing a channel (4) arranged inside the shell (3) for guiding a fluid, and comprising at least one inlet opening (6) for injecting the fluid into the channel (4), and at least one outlet opening (5) for releasing the fluid out of the channel (4) such that the at least one outlet opening (5) is arranged in a wall of the shell (3) for releasing the injected fluid to the outside of the implant (1), and
- optionally, filling the shell (3) with the filler composition.

All described embodiments of the invention have the advantage, that a breast implant and/or a method is provided, which allows for a less invasive possibility to apply a fluid, particularly a drug, to the tissue surrounding a breast implant. More particularly, the breast implant and the method of treatment according to the present invention allows for an effective and relatively non-invasive prevention or therapy of capsular contracture, particularly by application of collagenase enzyme treatment. Advantageously, the breast implant of the present invention may be applied for any of the usual purposes, i.e. for post-mastectomy breast reconstruction, for revision and reconstruction, and for aesthetic breast augmentation, in a similar way. Moreover, the breast implant of the present invention may also advantageously be applied for other purposes, for example, correction of male breast defects and deformities, particularly, as pectoral implants used for the reconstruction and the aesthetic repair of a man's chest wall.

### Example

The following example described in detail a method for application of a fluid including collagenase (preferably the FDA approved medicament sold at the day this invention is described under the brand Xiaflex®):
In a first step the inlet opening (6) or the injection port (7) are detected, for example by means of simply key them. Other techniques, such as x-ray, ultrasound or NMR, as described before, are suited as well. In a next step the part of the skin which needs to be penetrated by, for example, a syringe is provided with a local anesthetic in an amount suited to prevent pain from the patient. Preferably the inlet opening (6) or the injection port (7) are located in the lower right hand side quadrant of the breast.

By means of a syringe a liquid comprising collagenase enzyme is injected into the inlet opening (6) or injection port (7). The liquid comprises of consists of Xiaflex®.

Collagenase enzyme is allowed to distribute throughout the cannel (4) and will finally arrive at the outlet opening (5). The latter is located at the upper end of the implant (1).

Providing more of the fluid will allow the collagenase enzyme to pass the outlet opening (5) and due to gravity the fluid and hence collagenase enzyme will be distributed along the outer shell of the implant (1). This is beneath the disturbing collagen-fiber capsule.

Then one has to wait for an appropriate amount of time, which is in the frame of 24 hours.

In case symptoms of capsular contraction are not satisfactory alleviated the skilled physicist will gently squeeze the breast in order to open/break the capsule which causes undesired contraction. Such means can be successful because of the foregoing treatment with collagenase enzyme. Because the enzyme has weakened the collagen fiber capsule.

In cases of further pain and/or discomfort the treatment with Xiaflex® can be repeated. The dose of the drug can be equal or higher and it is within the skill of the physicist to apply suited amounts of the enzyme.

The present invention will be described in further detail with reference to the drawings from which further features, embodiments and advantages may be taken, and in which:

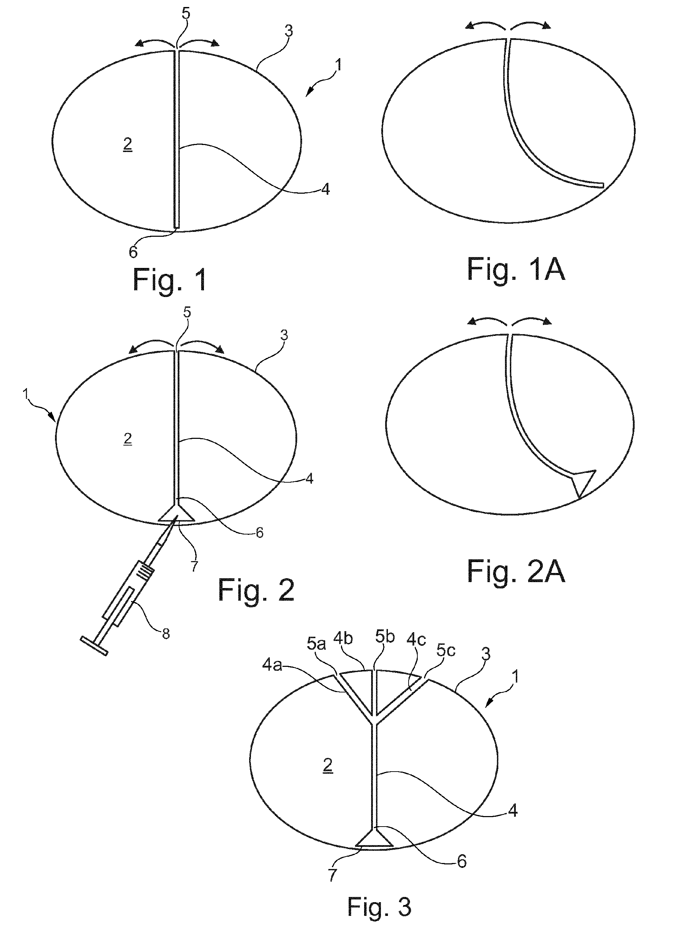

FIG 1 schematically illustrates a cross-sectional view of a breast implant of the present invention showing a first inventive embodiment;
FIG 1 a shows an alternative first inventive embodiment with a loopy channel (4);
FIG 2 schematically illustrates a cross-sectional view of a breast implant of the present invention showing a second inventive embodiment;
FIG 2a shows an alternative second inventive embodiment with a loopy channel (4)
FIG 3 schematically illustrates a cross-sectional view of a breast implant of the present invention showing a third inventive embodiment;
FIGs 4A and 4B schematically illustrate a cross-sectional view of a breast implant of the present invention emplaced in a female breast;
FIG 5 shows two possible positions of a breast implant (1), left hand side subglandular and right hand side subpectoral. Arrows indicate preferred position of the inlet (6) optionally including a port (7);
FIGs 6 and 6A show details of the inlet (6) with (6a) being the part of the inlet suited for multiple penetration and (6b) being the part of the inlet that connects the inlet (6) with the channel (4);
FIGs 7 and 7A show inlet (6) and its position in case the implant (1) includes another inlet (11) suited to expand the chamber (2) by filling in of filler composition.

### Detailed description of the drawings:

FIG 1 shows a breast implant (1) according to a first inventive embodiment of the present invention. The implant (1) comprises a shell (3) enclosing an internal chamber (2). Said chamber (2) is configured to be substantially filled with a filler composition. Particularly, the chamber (2) may be filled with filler composition prior to surgical emplacement or, alternatively, may also be filled after surgical emplacement. For such purpose, the breast implant (1) may be provided as a tissue expander and may comprise a filling port for injection of filler composition (not shown here). The chamber (2) of the breast implant (1) according to the present invention is fluid tight with regard to the exterior of the implant (1) and with regard to the interior of the channel (4). Accordingly, the breast implant (1) may be filled with any filler composition, particularly with saline solution, viscous silicone gel, and composite filler. The implant (1) comprises a channel (4) arranged inside the shell (3) for guiding a fluid, which is to be injected into the channel (4). For this purpose the channel (4) forms at its one end an inlet opening (6) for injecting the fluid into the channel (4). The fluid thus injected into channel (4) will be guided to the outlet opening (5) and will be released out of the channel (4). As may be immediately taken from FIG. 1 said outlet opening (5) is arranged in the upper wall of the shell (3) and thus the injected fluid will be released to the outside of the implant (1) surrounding said outlet opening (5).

FIG. 1 a shows an alternative embodiment with a loopy channel (4).

Turning to FIG. 2 the injection of such fluid using a syringe (8) is shown. For better understanding, the release of the fluid out of the outlet opening (5) is indicated with arrows. The embodiment of the implant (1) shown in FIG 2 departs from the embodiment shown in FIG. 1 in that the inventive implant (1), particularly the inlet opening (6) comprises an injection port (7). Said injection port (7) advantageously allows for simplified injection of the fluid and/or drug into the inlet opening (6). In both of the embodiments shown in FIG 1 and FIG 2 the implant (1) comprises only one singular unbranched channel (4) having one singular outlet opening (5) and one singular inlet opening (6). Such configuration advantageously allows a targeted delivery of the fluid and/or drug to a single desired target side. As may be immediately taken from either FIG 1 or FIG 2 the at least one inlet opening (6) and the at least one outlet opening (5) are located at distant, particularly opposite, sides of the implant (1). Here the implant (1) is shown in the orientation, which reflects its desired emplaced position in the female breast. Therefore, in the embodiments shown in FIG 1 and FIG 2 the outlet opening (5) is located at the dorsal side of the implant (1) and the inlet opening (6) is located at the ventral side of the implant (1) relating to its desired emplaced position in the human body. This particular configuration of the implant (1) advantageously allows release of the injected fluid and/or drug at the dorsal outlet opening (5) distant to the inlet opening (6), which - in its emplaced position in the human body - is usually relatively difficult to reach by standard methods of application of fluids and/or drugs. As particularly apparent from FIG. 2 the location of one outlet opening (5) at the dorsal side of the implant (1) advantageously promotes the distribution of the released fluid and/or drug around the shell (3) due to gravity, as indicated by the arrows.

FIG. 2a shows an alternative embodiment with a loopy channel (4).

Turning to FIG 3, the embodiment shown in said FIG 3 departs from the embodiment shown in FIG 2 in that the implant (1) comprises one singular channel (4), which is branched into three sub-channels (4a), (4b) and (4c) at the side of the channel (4) distant to the inlet opening (6). As may be immediately taken from FIG 3 in such embodiment the implant (1) thus has more than one outlet opening (5), particularly three outlet openings (5a), (5b) and (5c). In such configuration the fluid is injected into the one singular channel (4) having one singular inlet opening (6) and /or injection port (7) and is guided through the channel (4) being branched into the three distal sub-channels (4a), (4b) and (4c), each leading to one respective outlet opening (5a), (5b) and (5c). As apparent from FIG 3, such implant (1) having a branched channel (4) and comprising more than one outlet opening (5) each for releasing the fluid out of the desired channel (4), allows to release the fluid and/or drug simultaneously through all of the outlet openings (5a), (5b) and (5c). This particularly allows for simultaneous treatment of more than one target side and, moreover allows to simultaneously apply the fluid and/or drug to a greater target surface. Moreover, if one of the outlet openings (5a), (5b) and (5c) may be blocked or clogged, at least one, preferably two of the other remaining outlet openings may still be used for releasing the fluid out of the channel (4).

In all embodiments shown in FIG 1 to FIG 7 the channel (4) and the at least one inlet opening (6) is arranged completely inside the shell (3) and is thus not disturbing the outer surface of the implant (1). For injection of the fluid and/or drug the injection device, for example the syringe (8) as shown in FIG 2, is introduced through the shell (3) and into the inlet opening (6), particularly into the injection port (7), if present. Also the injection port (7) is arranged completely inside the shell (3). This advantageously allows for an even and undisturbed surface of the shell (3) of the implant (1) and, moreover protects the injection port (7), the inlet opening (6) and the channel (4) against unwanted damage.

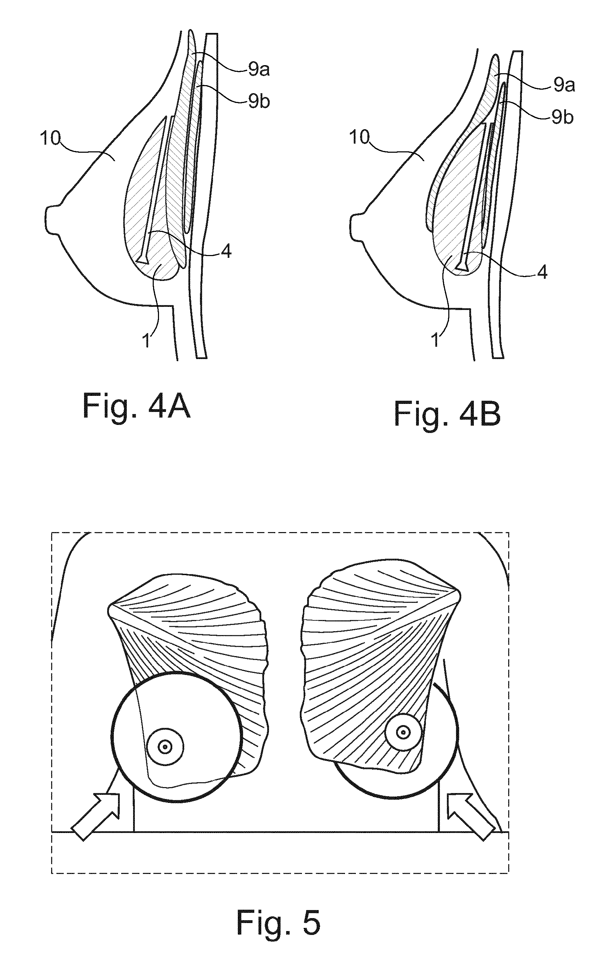

FIG 4A and FIG 4B and FIG 5 exemplarily show the inventive implant (1) in two different emplaced positions in the female breast. However, the implant (1) of the present invention may in general be emplaced according to any emplacement position and method known in the art, without adverting the intended purpose. For example, as may be seen from FIG 4A the implant (1) of the present invention may be emplaced subglandularily, i.e. the breast implant (1) is emplaced to the retromammary space, between the breast tissue, i.e. the mammary gland, (10) and the pectoralis major muscle (9a), which most approximates the plane of normal breast tissue, and affords the most aesthetic results. For such emplacement the implant (1) according to the present invention may even be preferred compared to standard implants known in the art as capsular contracture incidence rate is slightly greater with subglandular implantation.

Alternatively, the implant (1) according to the present invention may also be emplaced subpectorally, i.e. the breast implant (1) is emplaced beneath the pectoralis major muscle (9a), without releasing the inferior origin of the muscle proper. Total muscular coverage of the implant can be achieved by releasing the lateral muscles of the chest wall-either the serratus muscle or the pectoralis minor muscle (9b), or both-and suturing it, or them, to the pectoralis major muscle. In breast reconstruction surgery, the supectoral implantation approach effects maximal coverage of the breast implants. Still alternatively, the breast implant (1) according to the present invention may also be emplaced using other techniques known in the art, for example, subfascially, i.e. the breast implant is emplaced beneath the fascia of the pectoralis major muscle (9a); the subfascial position is a variant of the subglandular position for the breast implant; or subpectorally, i.e. the breast implant (1) is emplaced beneath the pectoralis major muscle (9a), after the surgeon releases the inferior muscular attachments, with or without partial dissection of the subglandular plane. Resultantly, the upper pole of the implant (1), where preferably at least one outlet opening (5) according to the present invention is located, is partially beneath the pectoralis major muscle (9a), while the lower pole according of the implant (1), where preferably an inlet opening (6) is located in the implant (1), is in the subglandular plane, and thus relatively easily accessible for injection of a fluid and/or drug.

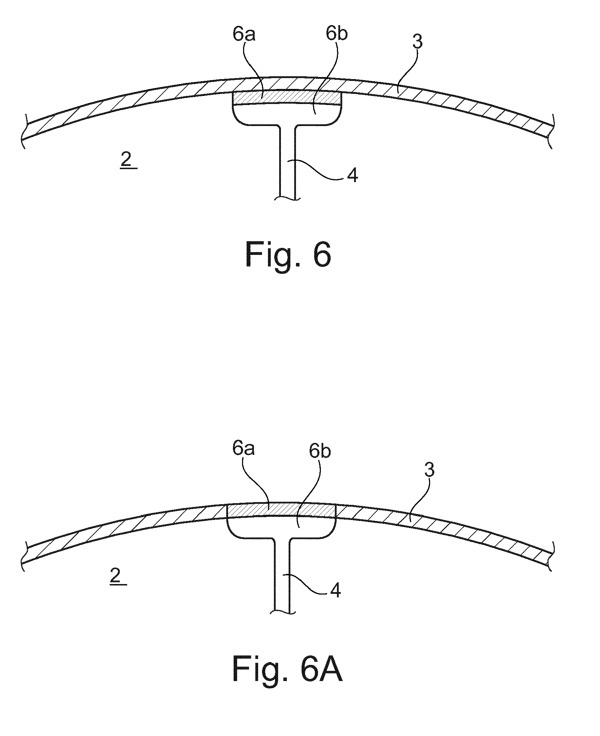

FIG 6 and 6A show details of the inlet port (6) which can be a two parts inlet port with a penetration part (6a) and a bridging part (6b). (6a) can be arranged inside the shell (3) or adjacent to the inner side of the shell (3). The part (6b) can be substituted by an injection port (7). In case of presence of an injection port one can also imagine that it is constructed similar as the inlet port shown in FIG 6a and 6a in a two part form with one part being an inlet opening, preferably suited for multiple penetration without rupture.

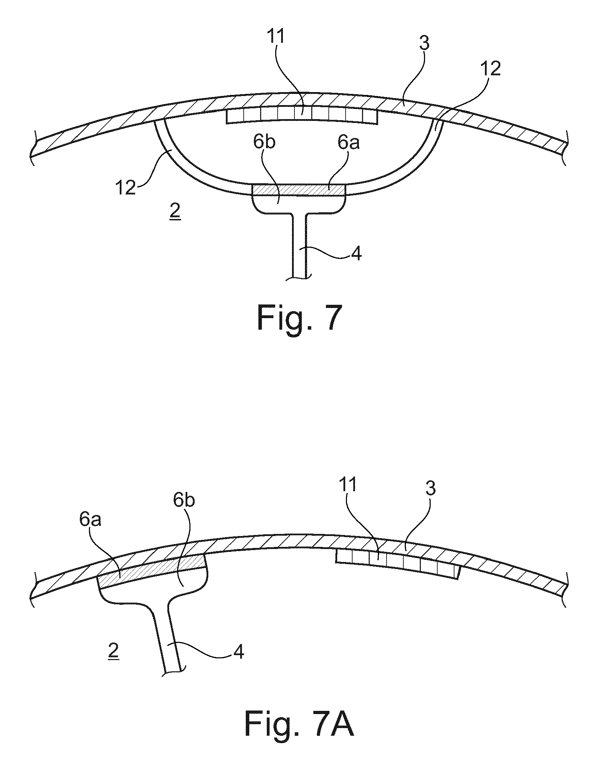

FIG 7 and FIG 7a show an inlet port (6) in a two part form comprising (6a) and (6b) in combination with an inlet for filling filler composition (11). Such further inlet (11) and injection port (6) can be arranged as show in FIG 7 (sequentially) or in FIG 7a (parallel). In case of the FIG 7 the syringe for application of the fluid comprising drug will penetrate both, (11) and (6) for ending up in the channel (4).

The features of the present invention disclosed in the specification, the claims and/or the figures may both separately and in any combination thereof be material for realizing the invention in various forms thereof.

### List of reference numerals

- 1: breast implant
- 2: chamber configured to be substantially filled with filler composition
- 3: shell
- 4: channel
- 5: outlet opening
- 6: inlet opening
- 6a: inlet opening (penetration part)
- 6b: inlet opening (bridging part)
- 7: injection port
- 8: syringe
- 9a: Pectoralis Major
- 9b: Pectoralis Minor
- 10: breast tissue
- 11: inlet for filling filler composition
- 12: mount for inlet opening (6)

## Claims

1. A breast implant (1) comprising
a shell (3) enclosing an internal chamber (2) configured to be substantially filled with a filler composition,
wherein the implant (1) comprises
- a channel (4) arranged inside the shell (3) for guiding a fluid, and comprising at least one inlet opening (6) for injecting the fluid into the channel (4), and at least one outlet opening (5) for releasing the fluid out of the channel (4),
and wherein the at least one outlet opening (5) is arranged in a wall of the shell (3) for releasing the injected fluid to the outside of the implant (1).

2. The implant (1) according to claim 1, wherein the at least one inlet opening (6) is arranged inside the shell (3).

3. The implant (1) according to any one of claims 1 or 2, wherein the inlet opening (6) comprises an injection port (7).

4. The implant (1) according to claim 3, wherein the injection port (7) is arranged inside the shell (3).

5. The implant (1) according to any one of claims 1 to 4, wherein the implant (1) comprises one singular unbranched channel (4) having one singular outlet opening (5) and one singular inlet opening (6).

6. The implant (1) according to any one of claims 1 to 4, wherein the implant (1) comprises a plurality of channels (4).

7. The implant (1) according to any one of claims 1 to 6, wherein the at least one inlet opening (6) and the at least one outlet opening (5) are located at opposite sides of the implant (1).

8. The implant (1) according to any one of claims 1 to 7, wherein the at least one outlet opening (5) comprises a closing mechanism.

9. The implant (1) according to any one of claims 1 to 8, wherein the at least one channel (4) is formed as an integral part of the shell (3).

10. The implant (1) according to any one of claims 1 to 9, wherein the at least one channel (4) is at least partially attached to the inner surface of the shell (3).

11. The implant (1) according to any one of claims 1 to 9, wherein the at least one channel (4) has an inner diameter of about 0.1 to about 5.0 mm.

12. The implant (1) according to any one of claims 1 to 11, wherein the at least one inlet opening (6), particularly an injection port (7) if present, comprises a locating mechanism.

13. Method for the treatment or prevention of a disease, comprising at least the steps of:
- a) surgically emplacing a breast implant (1) according to any one of claims 1 to 12,
- optionally, filling the implant (1) with filling composition,
- b) detecting the at least one inlet opening (6), and
- c) injecting a fluid comprising an effective dose of a drug into at least one inlet opening (6) until said fluid comprising the drug is provided through at least one outlet opening (5) to an area at the outside of the implant (1) surrounding said at least one outlet opening (5).

14. Method according to claim 13, wherein the disease is capsular contracture, and wherein the drug comprises collagenase.

15. Method for manufacturing an implant (1) according to any one of claims 1 to 12, comprising the steps of:
- providing a shell (3) enclosing an internal chamber (2) configured to be sub-stantially filled with a filler composition,
- providing a channel (4) arranged inside the shell (3) for guiding a fluid, and comprising at least one inlet opening (6) for injecting the fluid into the channel (4), and at least one outlet opening (5) for releasing the fluid out of the channel (4) such that the at least one outlet opening (5) is arranged in a wall of the shell (3) for releasing the injected fluid to the outside of the implant (1), and
- optionally, filling the shell (3) with the filler composition.
